# EUROPEAN PATENT SPECIFICATION

(11) **EP 3 897 464 B1**
(45) Date of publication and mention of the grant of the patent: **01.02.2023**
(21) Application number: 19899102.8
(22) Date of filing: 16.12.2019
(51) Int. Cl.: A61F 2/30, A61F 2/36

(54) **STEMLESS EXPANDABLE HIP PROSTHESIS**
SCHAFTLOSE EXPANDIERBARE HÜFTPROTHESE
PROTHÈSE DE HANCHE EXTENSIBLE SANS TIGE

(30) Priority: 18.12.2018 TR 201819751
(43) Date of publication of application: 27.10.2021
(73) Proprietor: Dokuz Eylül Üniversitesi Rektörlügü, Alsancak, Izmir (TR)
(72) Inventor: HAVITÇIOGLU, Hasan, Urla/Izmir (TR); UZUN, Bora, Balçova/Izmir (TR)
(86) International application number: PCT/TR2019/051088
(87) International publication number: WO 2020/130985

(56) References cited:
- EP-A2- 1 987 785
- FR-A2- 2 653 660
- US-A- 5 702 481
- US-A1- 2004 030 395
- US-A1- 2006 004 459
- US-A1- 2013 204 387
- US-A1- 2013 231 665
- US-A1- 2017 079 798

## Description

### Technological Field:

The invention is related to an expandable hip prosthesis, to be used in patients whose femur head in hipbone is damaged due to any reason, which can replace the femur head, without any stem and configured in a manner such that it limits the motion within the trochanter. The closest prior art is document US 2013/204387 A1, which defines the preamble of claim 1.

### State of the Art:

Giving function to the joint with reduced motion or motionless by means of surgical intervention is named as "arthroplasty". Arthroplasty is performed by surgical intervention to the bones which form the joint by reshaping the bones or by applying prosthesis.

Malformations which increase due to various reasons in the hipbone lead to hip joint diseases. Various joint problems are seen such as osteoarthritis, avascular necrosis, rheumatoid arthritis, broken femur neck, other inflammatory arthritis, developmental dysplasia, Paget disease, arthrodesis (fusion) stroke, tumor, road accidents, soldier wounds etc. (Pramanik (2005))

The conditions that require hip arthroplasty:
✔ Total deformation of the joint due to degenerative arthritis or rheumatoid arthritis
✔ Some femur fracture (fragmental intercapsular fracture)
✔ Innate hipbone diseases
✔ Pathological fractures due to metastatic cancers

The hip arthroplasty is performed in two ways;
✔ Hemiarthroplasty, prosthesis is placed in the location of the femur head or acetabulum.
✔ Total Hip Arthroplasty (Total Hip prosthesis), prosthesis is placed both in the location of femur and acetabulum.

Degenerative and inflammatory diseases are being in the first place, many hip problems leads to pain and limitation in hip motions in patients. The main aim of the patients is to have a movable hip joint without pain. Total hip prosthesis (THP) operation is among the most successful operations of orthopedic surgery. Together with the developments in bio-engineering, more durable and long life implants are produced. Also the patients compared to the past require eliminating their pains and continuing their normal body functions instead of accepting pain and limited activities. For this reason, an increase is seen in number of THP's applied.

The aim in the total hip prosthesis (THP) is to; eliminate the pain and increase the functions. The successful surgical method applied within correct indications forms the basis in THP operations in order to obtain well-excellent results. In addition to these; by means of using an implant with appropriate material and design features the success in THP operations is increased. Today many prostheses with different material and design features are developed. The design and material features of the selected implant may allow for the prosthesis to be simple, producible, cheap and long lasting. Sufficient options shall be submitted to the surgeon and technical difficulties shall be eliminated. It shall require as least bone resection as possible during the operation. Kurtz et al (2007) have calculated on the basis of the national statistics institute data in the year 2011 that, more than 50 % of all arthroplasty operations would be made to patients under age 65. In the year 2030, they mentioned that 52 % of the TKP subjects would be young patients. These numbers show that, we will encounter with younger patients with more expectations and thus the prosthesis design and implant features for manufacturing long lasting implants shall be given required importance more than any time. An ideal prosthesis shall be as flexible as preventing stress shielding and shall be as rigid as providing primary stability. Surface cover of the prosthesis shall have sufficient durability which would not be subject to abrasion after its contact with the bone. The modular components shall provide optimal compatibility and primary stability simultaneously and shall be suitable to the surgical method. Today, the hip prosthesis used in total hip revision surgery has stem portion in long or short form which extend towards the femur body. In order to place this portion, the current solid bone structure is carved and bone as much as stem size is removed thereof and the stem portion is placed within the created gap. Thus more metal accumulation is caused by means of decreasing solid bone stock of the patient. Also in case the life of the prosthesis is expired or in case it is required to change the prosthesis due to any reason, the current prosthesis is removed and instead of this a larger sized prosthesis is placed. As a result of this revision process, more bone stock loss is experienced. The femoral stems have different types in terms of fixation and load transmission which are used widely.

It is possible to classify the femoral components as anatomic and flat according to the geometry of the stem distal. The anatomic stems imitate the angle of the femur neck with femur shaft on sagittal plane. The anatomic stems; has circular section and provide well distal fixation and leads to more micro-motion and power shielding effect. The success ratios concerning the anatomic stem are reported. (Garellick et al. 1999)

In order to decrease the femoral pain and protect the bone stock, short or metaphysis filling stem models are developed. As an example to these models Proxima (DePuy) model having HA covered porous surface can be given. (Ghera et al. 2009) The macro steps that it has in calcar portion enable transmitting the tangential loads on the implant to the bone as a compressive load. (Bilgen et al. (2011)

The hip prosthesis which are named as proximal loaded or cervico trochanteric are the prostheses which has a short stem at the femur neck however do not have a stem at the femur long axis. The following advantages are reported in various studies made with this type of prostheses:
✔ During implantation, against revision possibilities in the future, protecting the bone stock and soft tissue in large trochanteric and sub trochanteric regions (Khanuja et al., 2011; Morrey, 1989])
✔ Decrease of stress shielding formed with the metaphyseal bone resorption and diaphysial cortical hyperthrophy (Pipino, 2000),
✔ The stress accumulation on the end of a conventional distal fixed component causes pain. With a short stem this condition is eliminated. (Khanuja et al., 2011; Mai et al., 2010)
✔ The tensioning band effect of the iliotibial band enables compression forces in the proximal femur bot medially and laterally. (Leali et al., 2002), lateral cortex enables a powerful support as a second compression column (d'Imporzano & Pierannunzii, 2006)
✔ Transmits the load in a physiological manner from superior to inferior to the metaphysis. (Leali et al., 2002; Walker et al., 1999)
✔ Implantation method facilitates the minimal invasive approaches (McElroy et al., 2011).
✔ Any kind of bone stock can be acceptable due to a wide indication field (the surface arthroplasty risk index is over grade 3) and normal femoral morphology (Kim et al., 2011),
✔ Minimal invasive approach, less soft tissue damage and easier revision surgery due to undamaged bone stock under the small trochanter (d'Imporzano & Pierannunzii,2006).

In the literature researches made, as similar patent application to the invention in terms of form and function is not seen. Only the system named as "An expandable bone fixation screw during fixation of hip fractures" (Patent No: 2012/153338) which is a patent of our group is similar in terms of form however, this new invention comprises important differences in terms of aim of usage, field of usage and structure. Said system is a novel expandable bone fixation screw which is developed for increasing the rotational stability of the hip screws used in the treatment of the femur neck fractures particularly in osteoporotic bones. In this system, there is a screw-plate combination with an expansion feature by means of hinged bars which are passed through a plate and the neck extending to the body of the femur bone whose neck is broken but head is undamaged.

In another literature research made United States patent document No US 2,685,877 is encountered, in which, a femur head prosthesis with short-stem having a locking mechanism thereon is disclosed. By means of the locking mechanism on the stem, the prosthesis is prevented to be dislocated. The operation manner of the system is totally different.

In another literature research made United States patent document No US 2,718,228 is encountered, in which, a femur head prosthesis having a stem which is short and channeled thereon is disclosed.

In another literature research made United States patent document No US 4,129,903 is encountered, in which, a femurs head prosthesis which has a short stem, has 2 plates at its neck portion and lateral of the bone, has a screw hole on the rear plate and aims to prevent the rotation sent through this hole, is disclosed. It has two different versions such as single screwed and double screwed.

In another literature research made United States patent document No US 6,284,002 B1 is encountered, in which, a femur head prosthesis is disclosed which has a short stem and aims to prevent rotation and removal with a screw.

In another literature research made United States patent document No US 2003/0187514 A1 is encountered, in which, a femur head prosthesis is disclosed which has an inclined and conical short stem.

In another literature research made United States patent document No US 2008/0009951 A1 is encountered, in which, a short-stem prosthesis system which is directed for reforming the femur head and neck totally or partially, can be fixed by means of a screw, is disclosed.

In another literature research made United States patent document No US 2008/0004710 A1 is encountered, in which, a two-component system having a cover fitted on the femur neck after the femur head is shaved and an acetabular socket placed thereon is disclosed.

In another literature research made United States patent document No US 8,470,049 B2 is encountered, in which, a hip prosthesis with wide, short and elliptical structure is disclosed. Its fixation by means of a screw is anticipated in order to increase the stability of the system.

In another literature research made United States patent document No US 2013/0060347 is encountered, in which short-stem prosthesis with a gradual structure is disclosed.

As a result a novel hip prosthesis is required in which the present state of the art is exceeded, the disadvantages are eliminated.

### Brief Description of the Invention:

The invention is a novel hip prosthesis in which the present state of the art is exceeded, the disadvantages are eliminated and additional features are included.

The aim of the invention is to provide a novel system for patients whose femur head is damaged, which enables protecting more bone stock compared to other prostheses while it allows for maximum motion of the femur and at the same time increases the rotational stability.

The present invention in order to realize all aims occurred due to the abovementioned and below described detailed description, is an expandable hip prosthesis as defined in claim 1.

Another preferred embodiment of the invention comprises a rotation point which enables upward motion of the lower plate by means of rotating the grooved shaft via a hand tool and thus enables fixation at the trochanter neck of the hip bone after the levers are expanded, is positioned on the bottom portion of the grooved shaft.

Another preferred embodiment of the invention comprises slot connection ends which enables the movable connection of the slot and the sphere headed levers to the sphere headed levers.

Another preferred embodiment of the invention comprises sphere connection ends which engages spherical headed levers with the slot connection ends in the sphere slot and the slot and the spherical headed levers and engages spherical headed levers with the sphere slots.

Another preferred embodiment of the invention comprises sphere locking rings which are disposed on the front portion of the sphere slots, prevents displacement of the sphere connection ends from the sphere slots.

Another preferred embodiment of the invention comprises a roller which enables fixing the upper plate on the upper portion of the grooved shaft in a stable manner.

Another preferred embodiment of the invention comprises a neck which is disposed on the upper end of the grooved shaft (15) and enables placing the spherical head.

Another preferred embodiment of the invention comprises a stopper which prevents the lower plate's closing the expanded levers by means of moving in a downward manner at the bottom portion of the lower plate when the hip prosthesis) is within the hip bone.

Another preferred embodiment of the invention comprises a case which surrounds the hip prosthesis and is removed from the prosthesis during its application to the hip bone.

Another preferred embodiment of the invention comprises a hand tool which rotates the grooved shaft from the rotation point positioned at the bottom portion.

Another preferred embodiment of the invention comprises movable joint sides consisting of the combination of sphere connection end and slot connection ends included in the levers.

### Description of Figures:

The invention will now be described with reference to the attached drawings, thus the features of the invention will be clearly understood. However, the aim of this is not limiting the invention with particular embodiments. On the contrary it is aimed to cover all alternatives and amendments that would include the field of the invention described in the attached claims. It is to be understood that the details shown are illustrated for the sake of only clarifying the preferred embodiments of the present invention and forming the methods, and also for providing the most useful and apparent description of the rules of the invention and conceptual features. In these drawings;
- Figure - 1: is an upper perspective view of the inventive hip prosthesis.
- Figure - 2: is a lower perspective view of the inventive hip prosthesis.
- Figure - 3: is a view of the inventive hip prosthesis where it is applied into the femur bone.
- Figure - 4A: is a transparent view of the inventive hip prosthesis with case.
- Figure - 4B: is a perspective view of the inventive hip prosthesis with its case.
- Figure - 5: is a view of the hand tool used in the application of the inventive hip prosthesis.

The figures clarifying this invention are enumerated as shown in the attached drawing and they are given with their names herein below.

### Description of the References:

**1.** Hip prosthesis
**2.** Spherical head
**3.** Upper plate
**4.** Lower plate
**5.** Sphere slot
**6.** Sphere locking ring
**7.** Roller
**8.** Slot and spherical headed lever
**9.** Spherical headed lever
**10.** Neck
**11.** Rotation point
**12.** Stopper
**13.** Case
**14.** Hand tool
**15.** Grooved shaft
**16.** Joint sides

### Description of the Invention:

In this detailed description, the inventive hip prosthesis (1) is only described for clarifying the subject matter in non-limiting manner. In the description an expandable hip prosthesis (1), to be used in patients whose femur head (F) in hipbone (K) is damaged due to any reason, which can replace the femur head (B), without any stem and configured in a manner such that it limits the motion within the trochanter, is disclosed.

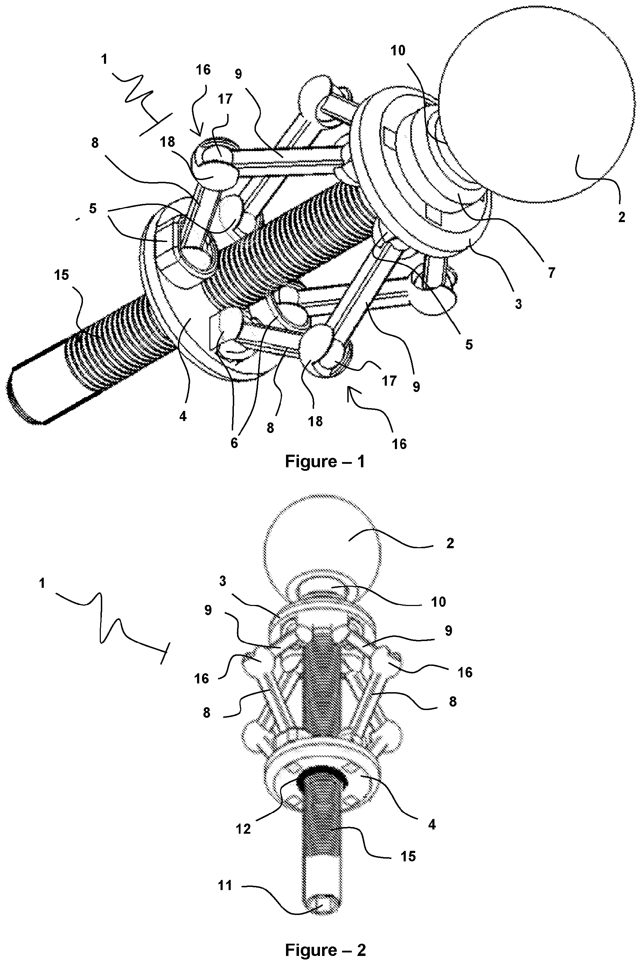

In Figure 1, the upper perspective view of the inventive hip prosthesis (1) is given. Accordingly the hip prosthesis (1) consists of the following; one grooved shaft (15), a spherical head (2) located on a neck (10) on the end portion of the grooved shaft (15), an upper plate (3) which is positioned on the lower portion of said neck (10) and remains stable, a roller (7) which prevents rotation of said upper plate (3) on the grooved shaft (15), a lower plate (4) which is located on the lower portion of the grooved shaft (15) and has a grooved center, sphere slots (5) which are located on the upper surface of the lower plate (4) and the lower surface of the upper plate (3), slot and spherical headed lever (8) which is attached within the sphere slots (5), a spherical headed lever (9) which is positioned in the sphere slots (5) disposed on the upper plate (3), sphere locking rings (6) which prevents displacement of the levers (8, 9) from the sphere slot (5), joint areas (16) which are the joint points of the levers (8, 9). The slot and spherical headed lever (8) comprises a sphere connection end (17) by means of which it can be located to the sphere slots (5) on the upper surface of the lower plate (4) and a slot connection end (18) which enables connection with the spherical headed lever (9). The spherical headed lever (9) comprises sphere connection ends (17) on both ends. Said sphere connection ends (17) establish connection with the slot connection end (18) at the end of the slot and spherical headed lever (8) and the sphere slots (5) on the lower surface of the upper plate (3). The slot connection ends (18) at the end of the slot and spherical headed lever (8) and the sphere connection ends (17) at the end of the spherical headed lever (9) are joined and form the joint areas (16).

In Figure 2, bottom perspective view of the inventive hip prosthesis (1). Accordingly the hip prosthesis (1) comprises a rotation point (11) at the bottom portion of the grooved shaft (15) in order to rotate the hip prosthesis, a stopper (12) which prevents reclosing of the lower plate (4) on its lower surface. Also it consists of a hand tool (14) which enables the rotation of the grooved shaft (15) through the rotation point (11).

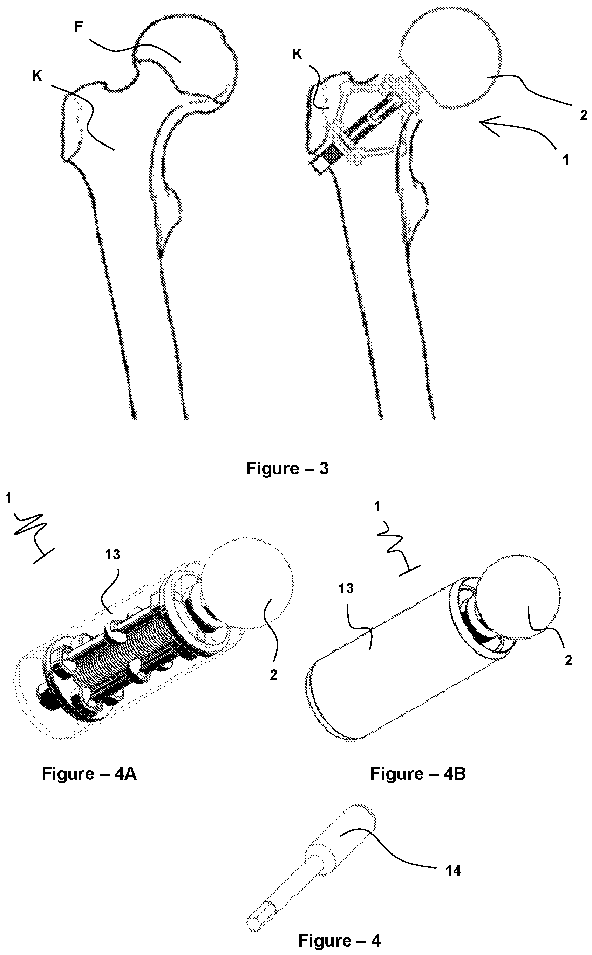

In Figure 3, the inventive hip prosthesis (1) is given in which it is applied to the femur head (F) of the hip bone (K). Hip prosthesis (1) can replace femur head (F) as can be seen in the figure in patients whose femur head (F) in the hip bone (K) due to any reasons. Hip prosthesis (1) comprises a spherical head (2) which is appropriate to the anatomic structure of the femur head (F).

In Figure 4A, a transparent view of the inventive hip prosthesis (1) comprising a case (13), in Figure 4B non-transparent perspective view is given. Said case (13) surrounds the hip prosthesis (1).

In Figure 5, the view of the hand toll (14) which enables rotating the grooved shaft (15) through the rotation point (11) is shown.

The inventive hip prosthesis (1) after the osteotomy and drilling process on the femur neck (F), is placed into the bone (K) with the case (13) which surrounds the whole system. Then the case (13) is removed. The lower plate (4) moves in an upward manner over the grooved shaft (15) after the grooved shaft (15) is rotated through the rotation point (11) by means of a hand tool (14). At this moment, the upper plate (3) remains stable since it is positioned with the roller (7) on the grooved shaft (15). While the lower plate (4) moves in an upward manner on the grooved shaft (15), the levers (8, 9) positioned in the sphere slots (5) located on the lower plate (4) and the upper plate (3) are oriented outside the joint (16) and thus the system expands. Together with the expansion of the system, the fixation at the trochanter neck increases and the motion is limited. After the sufficient fixation of the trochanter and femur neck, the spherical head (2) is placed on the neck (10).

### The sources /references used in preparing the description:

- Bilgen, Ö. F. Bilgen, S. Ermutlu, C. 2011. "Kalça protezlerinde malzeme ve tasarim özellikleri", TOTBiD Dergisi, 10(2):147-157
- Kurtz, S. Ong, K. Lau, E. Mowat, F. Halpern, M. 2007. "Projections of primary and revision hip and knee arthroplasty in the United States from 2005 to 2030.", J Bone Joint Surg [Am], 89:780-5.
- Pramanik, S. Agarwal, A.K. Rai. K.N. 2005 "Chronology of Total Hip Joint Replacement and Materials Development", Trends Biomater. Artif. Organs, Vol 19(1) pp15-26
- Park, M.S. Choi, B.W. Kim, S.J. Park, J.H. 2003."Plasma spray-coated Ti femoral component for cementless total hip arthroplasty." J Arthroplasty,18:626-30.
- Aldinger, P.R. Jung, A.W. Pritsch, M. Breusch, S. Thomsen, M. Ewerbeck, V. et al. 2009. "Uncemented grit-blasted straight tapered titanium stems in patients younger than fifty-five years of age. Fifteen to twenty-year results." J Bone Joint Surg [Am], 91:1432-9.
- Garellick, G. Malchau, H. Regnér, H. Herberts, P. 1999. "The Charnley versus the Spectron hip prosthesis: radiographic evaluation of a randomized, prospective study of 2 different hip implants." J Arthroplasty. 14:414-25.
- Aldinger, P.R. Thomsen, M. Mau, H. Ewerbeck, V. Breusch, S.J. 2003."Cementless Spotorno tapered titanium stems: excellent 10-15-year survival in 141 young patients". Acta Orthop Scand;74:253-8.
- Ghera,. S, Pavan, L. 2009. "The DePuy Proxima hip: a short stem for total hip arthroplasty. Early experience and technical considerations." Hip Int, 19:215-20.
- Khanuja, H.S.; Vakil, J.J., Goddard, M.S., & Mont, M.A. (2011). "Cementless Femoral Fixation in Total Hip Arthroplasty. " The Journal of Bone and Joint Surgery, AmericanVol.93, No.5, (March 2011), pp. 500-509
- Morrey, B.F. (1989). "Short-stemmed uncemented femoral component for primary hip arthroplasty." Clinical Orthopedics and Related Research, Vol.249, (December 1989), pp. 169-175
- Pipino F. (2000). The bone-prosthesis interaction. Journal of Orthopaedics and Traumatology, Vol 1 No. 1, (December 2000), pp. 3-9
- Mai, K.T.; Verioti, C.A., Casey, K., Slesarenko, Y., Romeo, L., & Colwell C.W. Jr. (2010). "Cementless Femoral Fixation in Total Hip Arthroplasty." American Journal of Orthopedics, Vol.39, No.3, (March 2010), pp. 126-130
- Leali, A.; Fetto, J., Insler, H., & Elfenbein, D. (2002). "The effect of a lateral flare feature on implant stability." International Orthopaedics, Vol.26, No.3, (April 2002), pp. 166-169
- d'Imporzano, M. & Pierannunzii, L. (2006). "Minimally invasive total hip replacement". Journal of Orthopaedic Traumatology, Vol.7, (2006), pp. 42-50
- Walker, P.S.; Culligan, S.G., Hua, G., Muirhead Allwood S.K., & Bentley G. (1999). "The effect of lateral flare feature on uncemented hip stems." Hip International, Vol.9,(1999), pp. 71-80
- McElroy, M.J.; Johnson, A.J., Mont, M.A., & Bonutti P.M. (2011). "Short and Standard Stem Prostheses Are Both Viable Options for Minimally Invasive Total Hip Arthroplasty." Bulletin of the NYU Hospital for Joint Diseases, Vol.69(Suppl 1), (n.d.), pp. S68-76
- Kim, Y.H.; Kim, J.S., Park, J.W., & Joo, J.H. (2011). "Total hip replacement with a short metaphyseal-fitting anatomical cementless femoral component in patients aged 70 years or older " The Journal of Bone and Joint Surgery, British Vol.93, No.5, (May 2011), pp. 587-592

## Claims

1. An expandable hip prosthesis (1), to be used in patients whose femur head (F) is damaged due to any reason, which can replace the femur head (B), without any stem and configured in a manner such that it limits the motion within the trochanter; wherein the expandable hip prosthesis comprises:
- a grooved shaft (15),
- a lower plate (4) comprising a groove in the hole in the middle in the lower section of said grooved shaft (15),
- an upper plate (3) fixed to the upper section where the grooved section of the grooved shaft (15) is not present, and
- a spherical head (2) appropriate to the femur head (F) anatomy, **characterised in that** the expandable hip prosthesis further comprises: a plurality of sphere slots (5) which are disposed on a lower surface of the upper plate (3) and on an upper surface of the lower plate (4); a plurality of slot and spherical headed levers (8) and a plurality of spherical headed levers (9), wherein the plurality of sphere slots positioned on the lower surface of the upper plate (3) are engaged with the plurality of spherical headed levers (9) and the plurality of sphere slots positioned on the upper surface of the lower plate (4) are engaged with the plurality of slot and spherical headed levers (8), wherein the plurality of spherical headed levers (9) and the plurality of slot and spherical headed levers (8) are engaged with each other at joint sides (16) in a manner such that they can move with respect to each other, and wherein the spherical head (2) is attached to a neck (10) on an upper portion of the grooved shaft (15).

2. The hip prosthesis (1) according to claim 1, comprising a rotation point (11) which enables upward motion of the lower plate (4) by means of rotating the grooved shaft (15) via a hand tool (14) and thus enables fixation at the trochanter neck of the hip bone (K) after the levers (8, 9) are expanded, is positioned on the bottom portion of the grooved shaft (15).

3. The hip prosthesis (1) according to claim 1, comprising slot connection ends (18) which enable the movable connection of the slot and the sphere headed levers (8) to the sphere headed levers (9).

4. The hip prosthesis (1) according to claim 1, comprising sphere connection ends (17) which engage spherical headed levers (9) with the slot connection ends (18) in the sphere slot (5) and slot and the spherical headed levers (9) and engage spherical headed levers (8) with the sphere slots (5).

5. The hip prosthesis (1) according to claim 1, comprising sphere locking rings (6) which are disposed on the front portion of the sphere slots (5), preventing displacement of the sphere connection ends (17) from the sphere slots (5).

6. The hip prosthesis (1) according to claim 1, comprising a roller (7) which enables fixing the upper plate (3) on the upper portion of the grooved shaft (15) in a stable manner.

7. The hip prosthesis (1) according to claim 1, comprising a neck (10) which is disposed on the upper end of the grooved shaft (15) and enables placing the spherical head (2).

8. The hip prosthesis (1) according to claim 1, comprising a stopper (12) which prevents the lower plate (4) from closing the expanded levers (8, 9) by means of moving in a downward manner at the bottom portion of the lower plate (4) when the hip prosthesis (1) is within the hip bone (K).

9. The hip prosthesis (1) according to claim 1, comprising a case (13) which surrounds the hip prosthesis (1) and is removed from the prosthesis (1) during its application to the hip bone (K).

10. The hip prosthesis (1) according to claim 1, comprising a hand tool (14) which rotates the grooved shaft (15), from the rotation point (11) positioned at the bottom portion.

11. The hip prosthesis (1) according to claim 1, wherein the movable joint sides (16) consist of the combination of sphere connection end (17) and slot connection ends (18) included in the levers (8, 9).

## Patentansprüche

1. Expandierbare Hüftprothese (1) zur Verwendung bei Patienten, deren Femurkopf (F) aus irgendeinem Grund beschädigt ist, die den Femurkopf (B) ersetzen kann, ohne jeden Stiel und so konfiguriert, dass sie die Bewegung innerhalb des Trochanters begrenzt; wobei die expandierbare Hüftprothese umfasst:
- eine gerillte Welle (15),
- eine untere Platte (4), die in der Mitte des unteren Abschnitts der gerillten Welle (15) eine Rille in der Bohrung umfasst,
- eine obere Platte (3), die an dem oberen Abschnitt befestigt ist, an dem der gerillte Abschnitt der gerillten Welle (15) nicht vorhanden ist, und
- einen Kugelkopf (2), der der Anatomie des Femurkopfes (F) entspricht,
**dadurch gekennzeichnet, dass** die expandierbare Hüftprothese weiterhin umfasst: eine Vielzahl von Kugelschlitzen (5), die auf einer unteren Fläche der oberen Platte (3) und auf einer oberen Fläche der unteren Platte (4) angeordnet sind; eine Vielzahl von Schlitz- und Kugelkopfhebeln (8) und eine Vielzahl von Kugelkopfhebeln (9), wobei die Vielzahl von Kugelschlitzen, die auf der unteren Fläche der oberen Platte (3) positioniert sind, mit der Vielzahl von Kugelkopfhebeln (9) in Eingriff sind und die Vielzahl von Kugelschlitzen, die auf der oberen Fläche der unteren Platte (4) positioniert sind, mit der Vielzahl von Schlitz- und Kugelkopfhebeln (8) in Eingriff sind, wobei die Vielzahl von Kugelkopfhebeln (9) und die Vielzahl von Schlitz- und Kugelkopfhebeln (8) an Gelenkseiten (16) so miteinander in Eingriff stehen, dass sie sich relativ zueinander bewegen können, und wobei der Kugelkopf (2) an einem Hals (10) an einem oberen Teil der gerillten Welle (15) angebracht ist.

2. Hüftprothese (1) nach Anspruch 1, umfassend
ein Drehpunkt (11), der eine Aufwärtsbewegung der unteren Platte (4) durch Drehen der gerillten Welle (15) mittels eines Handwerkzeugs (14) ermöglicht und somit eine Fixierung am Trochanterhals des Hüftbeins (K) nach dem Expandieren der Hebel (8, 9) ermöglicht, ist auf dem unteren Teil der gerillten Welle (15) positioniert.

3. Hüftprothese (1) nach Anspruch 1, umfassend
Schlitzverbindungsenden (18), die die bewegliche Verbindung der Schlitz- und Kugelkopfhebel (8) mit den Kugelkopfhebeln (9) ermöglichen.

4. Hüftprothese (1) nach Anspruch 1, umfassend
Kugelverbindungsenden (17), die mit den Schlitzverbindungsenden (18) in den Kugelschlitz (5) und den Schlitz- und die Kugelkopfhebel (9) eingreifen und mit den Kugelkopfhebeln (8) in die Kugelschlitze (5) eingreifen.

5. Hüftprothese (1) nach Anspruch 1, umfassend
Kugelverriegelungsringe (6), die auf dem vorderen Teil der Kugelschlitze (5) angeordnet sind und eine Verschiebung der Kugelverbindungsenden (17) aus den Kugelschlitzen (5) verhindern.

6. Hüftprothese (1) nach Anspruch 1, umfassend
eine Rolle (7), die es ermöglicht, die obere Platte (3) auf dem oberen Teil der gerillten Welle (15) auf stabile Weise zu befestigen.

7. Hüftprothese (1) nach Anspruch 1, umfassend
einen Hals (10), der am oberen Ende der gerillten Welle (15) angeordnet ist und das Aufsetzen des Kugelkopfes (2) ermöglicht.

8. Hüftprothese (1) nach Anspruch 1, umfassend
einen Stopper (12), der die untere Platte (4) daran hindert, die expandierten Hebel (8, 9) durch eine Abwärtsbewegung am unteren Teil der unteren Platte (4) zu verschließen, wenn sich die Hüftprothese (1) innerhalb des Hüftbeins (K) befindet.

9. Hüftprothese (1) nach Anspruch 1, umfassend
ein Gehäuse (13), das die Hüftprothese (1) umgibt und von der Prothese (1) während ihrer Anbringung am Hüftbein (K) entfernt wird.

10. Hüftprothese (1) nach Anspruch 1, umfassend
ein Handwerkzeug (14), das die gerillte Welle (15) vom Drehpunkt (11) aus, der sich am unteren Teil positioniert, dreht.

11. Hüftprothese (1) nach Anspruch 1, wobei
die beweglichen Gelenkseiten (16) bestehen aus einer Kombination von Kugelverbindungsenden (17) und Schlitzverbindungsenden (18), die in den Hebeln (8, 9) enthalten sind.

## Revendications

1. Prothèse de hanche expansible (1), pour être utilisée chez les patients dont la tête fémorale (F) est endommagée pour une raison quelconque, qui peut remplacer la tête fémorale (B), sans aucune tige et configurée de manière à limiter le mouvement à l'intérieur du trochanter; dans laquelle la prothèse de hanche expansible comprend:
- un arbre rainuré (15),
- une plaque inférieure (4) comprenant une rainure dans le trou au milieu dans la section inférieure dudit arbre rainuré (15),
- une plaque supérieure (3) fixée à la section supérieure où la section rainurée de l'arbre rainuré (15) n'est pas présente, et
- une tête sphérique (2)
appropriée à l'anatomie de la tête fémorale (F),
**caractérisé en ce que** la prothèse de hanche expansible comprend en outre: une pluralité de fentes sphériques (5) qui sont disposées sur une surface inférieure de la plaque supérieure (3) et sur une surface supérieure de la plaque inférieure (4); une pluralité de leviers à fente et à tête sphérique (8) et une pluralité de leviers à tête sphérique (9), dans lequel la pluralité de fentes sphériques positionnées sur la surface inférieure de la plaque supérieure (3) sont engagées avec la pluralité de leviers à tête sphérique (9) et la pluralité de fentes sphériques positionnées sur la surface supérieure de la plaque inférieure (4) sont engagées avec la pluralité de leviers à fente et à tête sphérique (8), dans lequel la pluralité de leviers à tête sphérique (9) et la pluralité de leviers à fente et à tête sphérique (8) sont en prise les uns avec les autres au niveau des côtés de jonction (16) de manière à ce qu'ils peuvent se déplacer les uns par rapport aux autres, et dans lequel la tête sphérique (2) est fixée à un col (10) sur une partie supérieure de l'arbre rainuré (15).

2. Prothèse de hanche (1) selon la revendication 1, comprenant un point de rotation (11) qui permet un mouvement vers le haut de la plaque inférieure (4) au moyen de la rotation de l'arbre rainuré (15) via un outil à main (14) et qui permet ainsi la fixation au niveau du col du trochanter de l'os iliaque (K) après que les leviers (8, 9) sont déployés, est positionné sur la partie inférieure de l'arbre rainuré (15).

3. Prothèse de hanche (1) selon la revendication 1, comprenant des extrémités de connexion à fente (18) qui permettent la connexion mobile de la fente et des leviers à tête sphérique (8) aux leviers à tête sphérique (9).

4. Prothèse de hanche (1) selon la revendication 1, comprenant des extrémités de connexion sphériques (17) qui engagent des leviers à tête sphérique (9) avec les extrémités de connexion à fente (18) dans la fente sphérique (5) et la fente et les leviers à tête sphérique (9) et qui engage les leviers à tête sphérique (8) avec les fentes sphériques (5).

5. Prothèse de hanche (1) selon la revendication 1, comprenant des anneaux de verrouillage sphériques (6) qui sont disposés sur la partie avant des fentes sphériques (5), empêchant le déplacement des extrémités de connexion sphériques (17) des fentes sphériques (5).

6. Prothèse de hanche (1) selon la revendication 1, comprenant un rouleau (7) qui permet de fixer de manière stable le plateau supérieur (3) sur la partie supérieure de la tige rainurée (15).

7. Prothèse de hanche (1) selon la revendication 1, comprenant un col (10) qui est disposé sur l'extrémité supérieure de la tige rainurée (15) et qui permet de placer la tête sphérique (2).

8. Prothèse de hanche (1) selon la revendication 1, comprenant une butée (12) qui empêche la plaque inférieure (4) de fermer les leviers déployés (8, 9) en se déplaçant vers le bas de la partie inférieure de la plaque inférieure (4) lorsque la prothèse de hanche (1) est à l'intérieur de l'os coxal (K).

9. Prothèse de hanche (1) selon la revendication 1, comprenant un boîtier (13) qui entoure la prothèse de hanche (1) et qui est retiré de la prothèse (1) lors de son application sur l'os iliaque (K).

10. Prothèse de hanche (1) selon la revendication 1, comprenant un outil à main (14) faisant tourner l'arbre rainuré (15), à partir du point de rotation (11) positionné à la partie inférieure.

11. Prothèse de hanche (1) selon la revendication 1, dans laquelle les côtés d'articulation mobiles (16) consistent en la combinaison d'une extrémité de connexion sphérique (17) et d'extrémités de connexion à fente (18) incluses dans les leviers (8, 9).
